# EUROPÄISCHE PATENTSCHRIFT

(11) **EP 2 890 702 B1**
(45) Veröffentlichungstag und Bekanntmachung des Hinweises auf die Patenterteilung: **24.08.2016**
(21) Anmeldenummer: 13780050.4
(22) Anmeldetag: 28.08.2013
(51) Int. Cl.: C07F 9/44, C07F 9/655, C08K 3/00

(54) **VERFAHREN ZUR HERSTELLUNG VON PHOSPHORHALTIGEN FLAMMSCHUTZMITTELN**
METHOD FOR PRODUCING PHOSPHORUS-CONTAINING FLAME RETARDANTS
PROCÉDÉ DE PRÉPARATION D'IGNIFUGEANTS PHOSPHORÉS

(30) Priorität: 29.08.2012 AT 9442012
(43) Veröffentlichungstag der Anmeldung: 08.07.2015
(73) Patentinhaber: Metadynea Austria GmbH, 3500 Krems (AT)
(72) Erfinder: ZICH, Thomas, A-4020 Linz (AT); FREIDL, Fritz, Johann, A-3500 Krems (AT); MEHOFER, Bernadette, 3500 Krems (AT); DÖRING, Manfred, 76744 Wörth am Rhein (DE); CIESIELSKI, Michael, 06217 Merseburg (DE); BURK, Bettina, 64293 Darmstadt (DE)
(74) Vertreter: Hoyng Rokh Monegier LLP
(86) Internationale Anmeldenummer: PCT/AT2013/050167
(87) Internationale Veröffentlichungsnummer: WO 2014/032070

(56) Entgegenhaltungen:
- CN-A- 102 190 814
- JP-A- 8 012 692
- US-A1- 2003 120 021

## Beschreibung

Die Erfindung betrifft ein neues Verfahren zur Herstellung von phosphorhaltigen Verbindungen, die als Flammschutzmittel wirksam sind.

Phosphorverbindungen sind seit Langem als Flammschutzmittel bekannt. Darunter finden sich in den letzten Jahren häufig auch 9,10-Dihydro-9-oxa-10-phosphaphen-anthren-10-on oder -oxid (DOPO), das erstmals von Sanko Chemical Co. Ltd. in DE 20 34 887 beschrieben wurde, und verschiedene Derivate davon. Deren Flammschutzeigenschaften scheinen darauf zu beruhen, dass sie beim Erhitzen phosphorhaltige Radikale freisetzen (siehe z.B. Schäfer et al., J. Appl. Polym. Sci. 105(2), 685-696 (2007)).

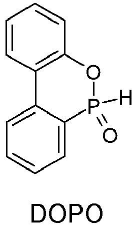

Auch Derivate von Diphenylphosphinoxid (DPhPO) und Diphenylphosphit (DPhOPO) sind als Phosphorverbindungen mit flammhemmender Wirkung und ähnlichem Wirkmechanismus bekannt.

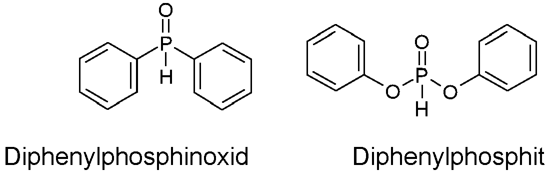

Andererseits zählen Melamin- und Guanamin-Derivate zu den bekannten stickstoffhaltigen Verbindungen mit Flammschutzwirkung, 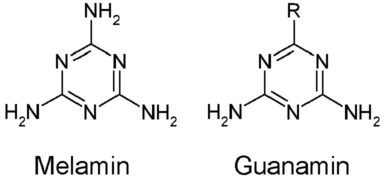 weswegen Versuche unternommen wurden, solche phosphorhaltige und stickstoffhaltige Verbindungen in Flammschutzmitteln zu kombinieren. Dabei wurde auch versucht, kovalente Verbindungen zwischen solchen Molekülgruppen herzustellen.

In US 2003/120021 A1, entspricht US 6.797.821 B2, und US 2005/0004339 A1 von Wang et al. werden Härter für Epoxyharze bzw. damit gehärtete Epoxyharze beschrieben, die jeweils eine oder mehrere DOPO- bzw. Diarylphosphinoxid-Gruppen, kovalent an stickstoffhaltige Moleküle gebunden, darunter auch Melamin, Methyl- und Phenylguanamin, enthalten und der folgenden Formel entsprechen:

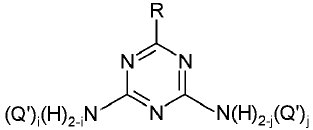

Darin kann Q' unter anderem ein DOPO- oder DPhPO-Rest sein, R kann NH₂, CH₃ oder Phenyl sein, und i und j stehen jeweils für 0, 1 oder 2. Hergestellt werden in den Synthesebeispielen 13 und 14 beider zitierter Anmeldungen allerdings nur Produkte der obigen Formel, worin i = 1 und j = 0 ist, also einfach phosphoryliertes Melamin bzw. Guanamin, und zwar durch Umsetzung von 1 mol DOPO-Cl, d.h. 10-Chlor-9,10-dihydro-9-oxa-10-phosphaphenanthren-10-on, bzw. 1 mol DPhPO-Cl, d.h. Diphenylphosphorylchlorid, mit 1 mol Melamin unter Erhitzen auf rund 170 °C. Allgemein erwähnt wird auch die analoge Synthese von Verbindungen mit mehr als einer phosphorhaltigen DOPO- oder DPhPO-Gruppe durch Umsetzung von i+j mol Q'Cl mit 1 mol Melamin oder Guanamin, ohne jedoch ein konkretes Verfahren anzuführen.

Die Erfinder haben im Verlauf ihrer Forschungen herausgefunden, dass diese analoge Umsetzung von bis zu 4 mol DOPOCI oder DPhPOCl mit 1 mol Melamin oder Guanamin nicht zu den gewünschten Produkten führen kann, wenn i und/oder j = 2 sein soll(en), und insbesondere nicht, wenn Melamin nur an zwei seiner drei Aminogruppen derivatisiert werden soll. Aufgrund der Reaktionsträgheit des Wasserstoffs einer bereits einfach mit der jeweiligen Phosphorverbindung derivatisierten Aminogruppe, also einer Gruppe -NHQ', kann es nämlich nicht einmal theoretisch gelingen, eine Aminogruppe zweifach mit Q' zu substituieren, ohne die Wasserstoffe der dritten Aminogruppe zu schützen, da -NH₂ gegenüber den Phosphorylchloriden deutlich reaktiver ist als -NHQ'.

Darüber hinaus umfasst das Verfahren von Wang et al. sehr lange Reaktionszeiten. So sind beispielsweise nach bereits beendeter Zugabe aller Reagenzien noch Rührzeiten von 16 h (für DOPO-Cl) bzw. 10 h (für DPhPO-Cl) erforderlich, um im Wesentlichen vollständigen Umsatz zu erzielen, obwohl bei Temperaturen von rund 170 °C gearbeitet wird.

Ziel der Erfindung war daher die Bereitstellung eines verbesserten Verfahrens zur Herstellung derartiger oder ähnlicher Verbindungen, mittels dessen diese Verbindungen in guten Ausbeuten, nach relativ kurzen Reaktionszeiten und im Wesentlichen ohne störende Nebenreaktionen erhältlich sind.

### OFFENBARUNG DER ERFINDUNG

Dieses Ziel erreicht die Erfindung durch Bereitstellung eines Verfahrens zur Herstellung von Verbindungen der nachstehenden Formel (I) 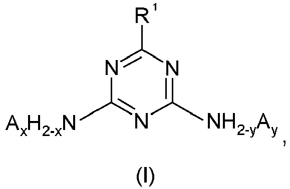 worin
- der Rest R¹ aus -NH₂, -NH_{2-z}A_{z} sowie einwertigen Alkyl- und Arylresten ausgewählt ist,
- die Reste A jeweils unabhängig voneinander aus den nachstehenden Phosphoryl-Resten DOPO-, DPhPO- und DPhOPO- ausgewählt sind 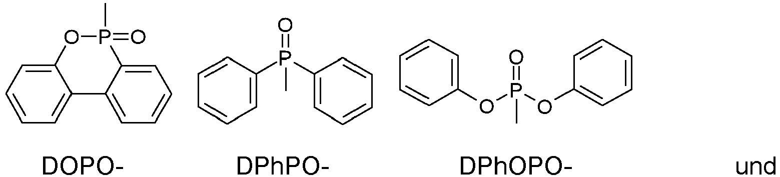
- die Indizes x, y, und z jeweils unabhängig voneinander für 0 oder 1 stehen, wobei zumindest einer der Indizes ≠ 0 ist;
   indem in einer ersten Stufe Melamin oder, wenn R¹ ein Alkyl- oder Arylrest ist, das entsprechende Alkyl- oder Arylguanamin mit einem oder mehreren der nachstehenden Phosphinylchloride DOP-Cl, DPhP-Cl und DPhOP-Cl 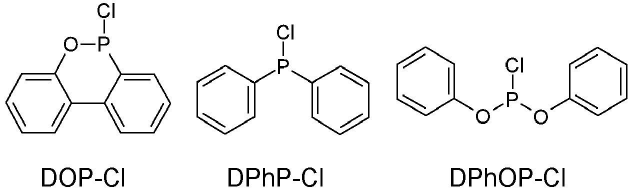 umgesetzt wird, um einen oder mehrere Phosphinylrest(e) an die Aminogruppe(n) des Melamins oder Guanamins zu binden, wonach in einer zweiten Stufe der/die gebundene(n) Phosphinylrest(e) durch Reaktion mit einem Oxidationsmittel zu dem/den entsprechenden Phosphorylrest(en) oxidiert wird/werden.
   Genauer gesagt wird in der ersten Stufe eine Verbindung der Formel (II) 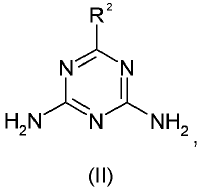 worin der Rest R² aus -NH₂ und einwertigen Alkyl- und Arylresten ausgewählt ist, d.h. Melamin oder ein Guanamin, mit einem oder mehreren der Phosphinylchloride DOP-Cl, DPhP-Cl und DPhOP-Cl zu einer oder mehreren Verbindungen der nachstehenden Formel (III) umgesetzt 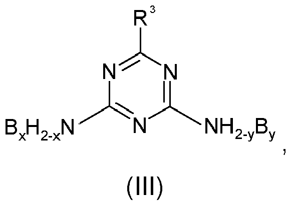 worin:
   - der Rest R³ aus -NH₂, -NH_{2-z}B_{z} sowie einwertigen Alkyl- und Arylresten ausgewählt ist,
   - die Reste B jeweils unabhängig voneinander aus den nachstehenden Phosphinyl-Resten DOP-, DPhP- und DPhOP- ausgewählt sind 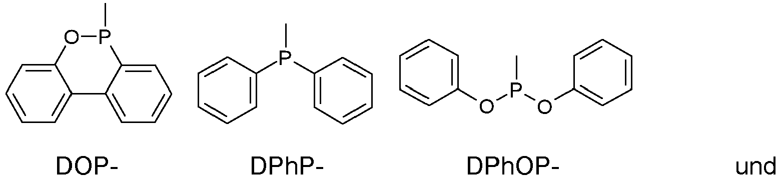
   - die Indizes x, y, und z wie zuvor definiert sind;
   wonach in der zweiten Stufe die Verbindung(en) der Formel (III) durch Reaktion mit dem Oxidationsmittel zu einer oder mehreren Verbindungen der Formel (I) oxidiert wird/werden.

Durch dieses neue Verfahren können nicht nur Verbindungen der Formel (I) in guten Ausbeuten und im Wesentlichen ohne Nebenreaktionen hergestellt werden, sondern es können auch mit dem jeweiligen phosphorhaltigen Rest dreifach substituierte Melamine problemlos hergestellt werden, und es sind auch ganz gezielt Gemische aus einfach und zweifach substituierten Aminotriazinen erhältlich, was die späteren Beispiele klar belegen. Darüber hinaus konnten die Reaktionszeiten gegenüber dem Verfahren von Wang et al. deutlich verkürzt werden, was speziell in Anbetracht der hohen Reaktionstemperaturen einen wesentlichen wirtschaftlichen Vorteil darstellt.

Ohne sich auf eine bestimmte Theorie beschränken zu wollen, wird angenommen, dass dies der im Vergleich zu den entsprechenden Phosphorylchloriden deutlich höheren Reaktivität der Phosphinylchloride zuzuschreiben ist. Überraschend waren dabei jedoch die nahezu quantitativen Ausbeuten bei der Herstellung der gewünschten Verbindungen. Gerade aufgrund der hohen Reaktivität der Phosphinylchloride wäre eigentlich anzunehmen, dass es vermehrt zu Nebenreaktionen kommt - speziell bei den bevorzugten hohen Reaktionstemperaturen von bis zu 200 °C. Das heißt, es war nicht zu erwarten, dass im Wesentlichen ausschließlich das jeweilige gewünschte Produkt mit je einem phosphorhaltigen Rest pro Aminogruppe gebildet wird, sondern vielmehr, dass daneben ein gewisser - wenn auch geringer - Anteil mit zwei phosphorhaltigen Resten an einer Aminogruppe entsteht. Dies war jedoch in keinem Fall zu beobachten. Somit weisen die im erfindungsgemäßen Verfahren eingesetzten Phosphinylchloride ausreichend hohe Reaktivität auf, um eine rasche Anbindung genau eines phosphorhaltigen Rests pro Aminogruppe zu ermöglichen, die aber nicht ausreicht, um die Aminogruppen zweifach zu substituieren, selbst wenn ein molarer Überschuss der Phosphinylchloride zum Einsatz kommt.

Weiters ist das im erfindungsgemäßen Verfahren zur Herstellung von DOPO-Derivaten eingesetzte Phosphinylchlorid DOP-CI ein industrielles Zwischenprodukt bei der Herstellung des gängigen Flammschutzmittels DOPO, wird daher in großen Mengen produziert und ist somit weitaus kostengünstiger und leichter erhältlich als das Phosphorylchlorid DOPO-Cl, das im Verfahren von Chun-Shan Wang zum Einsatz kommt.

Die einwertigen Alkyl- und Arylreste als Optionen der Reste R¹ bis R³ sind vorzugsweise -CH₃ oder -C₆H₅, also Methyl oder Phenyl, da diese gängige und leicht erhältliche Guanamine - Methylguanamin und Phenyl- oder auch Benzoguanamin - bilden. Das erfindungsgemäße Verfahren ist jedoch auf eine breite Palette von Substituenten am Diaminotriazin-Kern anwendbar, weswegen der Schutzumfang nicht auf diese beiden bevorzugten Reste beschränkt sein soll.

Die in der ersten Stufe erhaltenen phosphorhaltigen Zwischenprodukte sind weitgehend hydrolysestabil und können daher einfach isoliert werden, z.B. durch Einrühren in Wasser. Allerdings sind auch Eintopfreaktionen für beide Stufen möglich. Beides wird in den späteren Ausführungsbeispielen belegt.

Das Oxidationsmittel ist nicht speziell eingeschränkt, solange es zu keinen unerwünschten Nebenreaktionen der Reaktionspartner führt. In bevorzugten Ausführungsformen wird allerdings ein Peroxid eingesetzt, da etwaige Überschüsse einfach abzutrennen bzw. zu zerstören sind. Neben Wasserstoffperoxid, H₂O₂, kommen auch zahlreiche andere Peroxide und Hydroperoxide in Frage. Auch die literaturbekannte Oxidation von DOP zu DOPO mit Ozon ist möglich, lieferte aber in den Versuchen der Erfinder weniger gute Ergebnisse als Wasserstoffperoxid oder t-Butylhydroperoxid. Letzteres ist aufgrund seiner höheren Stabilität und daher einfacheren Handhabung gegenüber H₂O₂ zu bevorzugen.

Da bei der Reaktion zwischen Phosphinylchlorid und dem Aminotriazin HCl frei wird, wird die erste Stufe vorzugsweise in Gegenwart eines Säurefängers durchgeführt, um das Reaktionsgleichgewicht zur Produktseite hin zu verschieben. Der Säurefänger ist vorzugsweise 1-Methylimidazol, obwohl sich durchaus auch andere, dem Fachmann bekannte Verbindungen eignen wie etwa Ammoniak, Alkyl- und Arylamine und andere Stickstoffverbindungen, wie z.B. Triethylamin, Pyridin, Imidazol und dergleichen, oder andere Basen, wie z.B. Alkali- und Erdalkalimetallverbindungen. 1-Methylimidazol besitzt den großen Vorteil, dass sein Hydrochlorid bereits bei 75 °C schmilzt (während z.B. das nichtmethylierte homologe Imidazoliumchlorid einen Schmelzpunkt von 158-161 °C aufweist) und daher bei geeigneter Wahl des Lösungsmittels und der Reaktionstemperatur eine zweite flüssige Phase neben der Reaktionslösung bildet, die leicht abzutrennen ist.

Beide Stufen werden vorzugsweise in einem organischen Lösungsmittel durchgeführt, um die Homogenität der Reaktion und die Wärmeabfuhr sicherzustellen. Das Lösungsmittel ist nicht speziell eingeschränkt, solange es gegenüber den darin ablaufenden Reaktionen chemisch inert ist und die Ausgangsprodukte darin löslich oder zumindest dispergierbar sind. Es sollte jedoch in der ersten Stufe aus obigen Gründen einen deutlich über 75 °C liegenden Siedepunkt aufweisen, ausreichend gutes Lösungsvermögen für DOP-CI aufweisen und 1-Methylimidazoliumchlorid nicht lösen. Gemäß vorliegender Erfindung wird daher vorzugsweise ein relativ apolares, wasserfreies Lösungsmittel, noch bevorzugter ein aromatischer Kohlenwasserstoff, wie z.B. Benzol, Toluol, Xylol usw., insbesondere Toluol, eingesetzt. Vorzugsweise wird dasselbe Lösungsmittel sowohl für die Umsetzung des Aminotriazins mit dem Phosphinylchlorid als auch für die anschließende Oxidation eingesetzt. Es können jedoch auch unterschiedliche Lösungsmittel zum Einsatz kommen, beispielsweise Chloroform für die erste Stufe und Toluol für die Oxidation usw.

In bevorzugten Ausführungsformen der Erfindung fungiert in der ersten Stufe der Säurefänger gleichzeitig als Lösungsmittel, weswegen hier insbesondere 1-Methylimidazol sowohl als Säurefänger als auch als Lösungsmittel eingesetzt wird. In der zweiten Stufe werden Chloroform oder Toluol, aufgrund der Halogenfreiheit insbesondere Toluol bevorzugt.

Obwohl die Erfindung nicht darauf beschränkt ist, hat sich im Zuge der Forschungen der Erfinder gezeigt, dass die erste Stufe vorzugsweise bei einer Temperatur im Bereich von 100 bis 200 °C durchgeführt werden sollte, um kurze Reaktionszeiten, hohe Umsätze und gute Ausbeuten zu gewährleisten. In ähnlicher Weise haben sich für die zweite Stufe Temperaturen im Bereich von 50 bis 100 °C als vorteilhaft herausgestellt.

Im Schutzumfang der Erfindung liegen natürlich auch die unmittelbaren Verfahrensprodukte des erfindungsgemäßen Verfahrens, d.h. die so hergestellten Verbindungen der Formel (I).

Da das dreifach substituierte Melamin, d.h. 10.2,4,6-Tris(9,10-dihydro-9-oxa-10-oxo-10-phosphaphenanthren-10-ylamino)-1,3,5-triazin (DOPO₃-Mel): 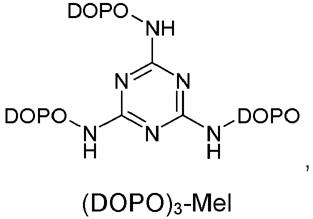 worin DOPO- dem Rest 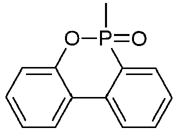 entspricht, eine neue chemische Verbindung darstellt, besteht ein weiterer Aspekt der Erfindung im Substanzschutz für dieses Produkt sowie in dessen Verwendung als Flammschutzmittel. In ersten Brandtests der Erfinder zeigt diese neue Verbindung nämlich ausgezeichnete Wirkung als Flammschutzmittel für Kunststoffe, insbesondere für Polystyrol und Epoxide.

### BEISPIELE

Die Erfindung wird nachstehend anhand von nichteinschränkenden Ausführungsbeispielen im Detail beschrieben.

### Beispiel 1

Herstellung von DOPO₃-Mel, d.h. 2,4,6-Tris(9,10-dihydro-9-oxa-10-oxo-10-phosphaphenanthren-10-ylamino)-1,3,5-triazin:

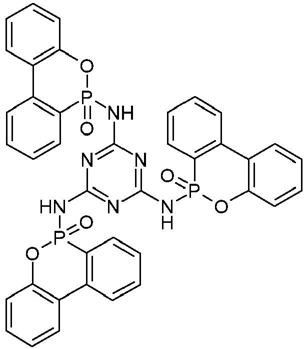

Stufe 1 - Herstellung von DOP₃-Mel, d.h. 2,4,6-Tris(9,10-dihydro-9-oxa-10-phosphaphenanthren-10-ylamino)-1,3,5-triazin:

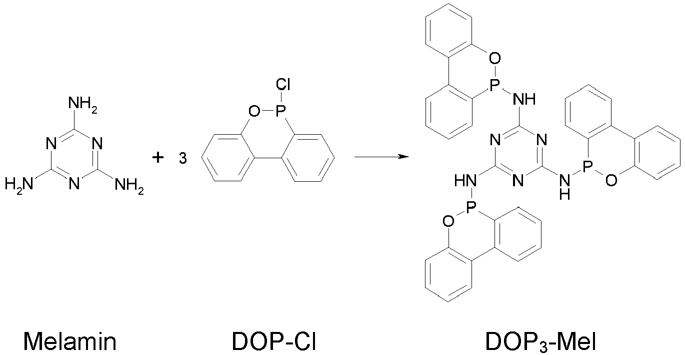

In einen mit Argon gefüllten 500-ml-Dreihalsrundkolben, der mit einem Innenthermometer, Tropftrichter, Rührer sowie einer Inertgasüberleitung ausgerüstet war, wurden 12,64 g (0,10 mol) Melamin sowie 82,1 g (1,00 mol) wasserfreies 1-Methylimidazol als Säurefänger und Lösungsmittel gefüllt und auf 100 °C erhitzt. Danach wurden 72,4 g (0,31 mol) DOP-CI bei ca. 100 °C unter Inertbedingungen geschmolzen und in den Tropftrichter gefüllt. Das DOP-CI wurde unter kräftigem Rühren im Verlauf von 1 h bei 100 °C zugetropft, wobei der Tropftrichter mit einem Heißluftföhn erhitzt wurde, um ein Erstarren des Reagens zu vermeiden. Anschließend wurde noch ca. 15 h lang bei 100 °C unter Argon gerührt, wonach der dickflüssige Kolbeninhalt in 500 ml Wasser eingerührt und der dabei ausgefallene körnige Feststoff mit einer Glasfritte abfiltriert wurde. Der Filterkuchen wurde dann 2x in jeweils 250 ml Wasser aufgeschlämmt und erneut abfiltriert. Anschließend wurde die Substanz gründlich mit 200 ml Aceton gewaschen und zuletzt mit n-Pentan gespült. Nach Trocknen im Luftstrom wurden 72,4 g (DOP)₃-Mel in quantitativer Ausbeute erhalten.

³¹P-NMR (101 MHz, DMSO-d₆): δ 66,2; 66,1; 65,9 ppm. ¹H-NMR (250 MHz, DMSO-d₆): δ 8,7-8,5 (d, 3 H, 3 NH-P); 8,25-8,15 (d, 6 H); 7,68-7,53 (t, 6 H); 7,50-7,40 (t, 3 H), 7,38-7,28 (t, 3 H), 7,27-7,17 (t, 3 H), 7,08-6,97 ppm (m, 3 H).

### Stufe 2 - Oxidation von DOP₃-Mel zu DOPO₃-Mel

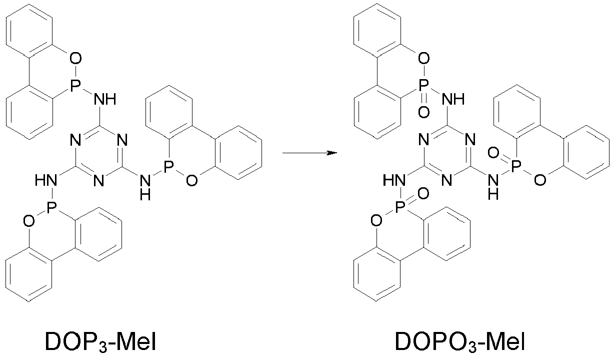

In einem mit Rührer, Innenthermometer und Tropftrichter ausgerüsteten 1-I-Dreihalskolben wurde das in Stufe 1 hergestellte (DOP)₃-Mel (72,4 g) bei 50 °C in 500 ml Chloroform eingerührt, wobei ein Teil in Lösung ging. Anschließend wurde das Gemisch mit einem Kühlbad auf ca. 12 °C abgekühlt und sofort mit der Zugabe einer 11 %igen Lösung von H₂O₂ (106,5 g, 34 mol) in Essigsäureethylester begonnen. Das Reagens wurde binnen etwa 1,5 h zugetropft, wobei kräftig gerührt und die Temperatur auf ca. 15 °C gehalten wurde. Dann wurde das Kühlbad entfernt und das Rühren noch 2 h lang fortgesetzt. Zur erhaltenen, trüben Lösung wurde wasserfreies Natriumsulfat zugesetzt, um das bei der Umsetzung entstandene Wasser abzutrennen. Nach dem Abfiltrieren des Trocknungsmittels wurde die Produktlösung bei 40 °C im Teilvakuum eingeengt, wobei sich eine zähe Substanz abschied. Nach Kühlen auf ca. 0 °C wurde dekantiert. Der verbliebene Feststoff wurde im Vakuum allmählich auf 230 °C erhitzt. Nach ca. 30 min bei dieser Temperatur, Abkühlen und Zerkleinern des so erhaltenen Rohprodukts wurde dieses in 150 ml Chloroform gelöst. Diese Lösung wurde unter kräftigem Rühren in 500 ml Diethylether getropft, wobei sich ein weißer, körniger Feststoff ausschied, der abfiltriert, mit Diethylether gewaschen und bei 60 °C im Vakuum getrocknet wurde. Auf diese Weise wurden 67 g (DOPO)₃-Mel erhalten (87,2 % d.Th.).

³¹P-NMR (101 MHz, DMSO-d₆): δ 6,86; 6,67; 6,30 ppm.

¹H-NMR (250 MHz, DMSO-d₆): δ 9,9-9,3 (3H, 3 NH-P); 8,40-8,22 (m, 3H); 8,18-8,00 (t, 6H); 7,78-7,58 (t, 3H), 7,55-7,35 (d, 6H), 7,35-7,16 ppm (m, 6H).
Elementaranalyse für C₃₉H₂₇N₆P₃O₆ (768,59 g/mol)
ber.: C 60,95; H 3,54; N 10,93;
gef.: C 60,52, H 3,71, N 10,80.

### Beispiel 2

Herstellung von DOPO₂-PhGuanamin, d.h. 2,4-Bis(9,10-dihydro-9-oxa-10-oxo-10-phosphaphenanthren-10-ylamino)-6-phenyl-1,3,5-triazin:

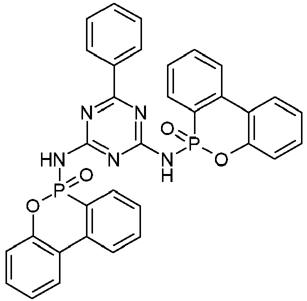

### Stufe 1 - Herstellung von DOP₂-PhGuanamin aus Phenylguanamin und DOP-CI

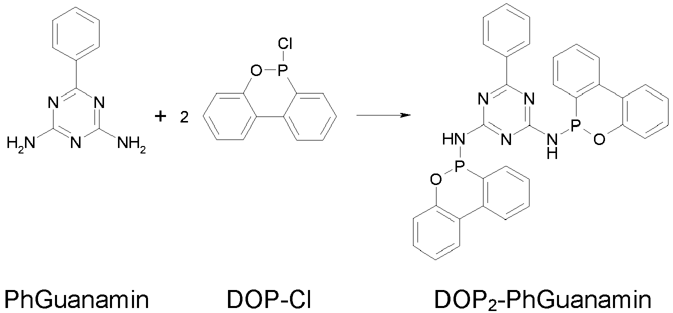

In einen mit Argon gefüllten und mit Rückflusskühler, Rührer und Tropftrichter ausgerüsteten Dreihalskolben wurden 6-Phenyl-1,3,5-triazin-2,4-diamin (Phenylguanamin; 33,1 g, 0,177 mol, 1 Äqu.) sowie 1-Methylimidazol (145 g, 1,77 mol, 10 Äqu.) gefüllt. Dann wurden 91,2 g (0,389 mol, 2,2 Äqu.) DOP-CI bei ca. 100 °C unter Inertbedingungen geschmolzen und in den Tropftrichter gefüllt. Das DOP-CI wurde unter kräftigem Rühren im Verlauf von 1 h bei 100 °C zugetropft, wobei ein Heißluftföhn benutzt wurde, um es flüssig zu halten. Das Reaktionsgemisch wurde noch 2 h lang bei dieser Temperatur gehalten und dann in 800 ml destilliertes Wasser eingerührt. Der ausgefallene Feststoff wurde abfiltriert und 3x mal mit Wasser sowie mit Aceton gewaschen. Dann wurde das Rohprodukt 2 h lang in 300 ml siedendem Toluol gerührt. Die noch heiße Suspension wurde filtriert, und der isolierte Feststoff wurde gründlich mit Toluol gewaschen und schließlich im Luftstrom getrocknet. Erhalten wurden so 97,24 g (0,1668 mol, 94,2 % d.Th.) (DOP)₂-PhGuanamin als weißer Feststoff.
Fp.: 276-282 °C (Toluol)
³¹P-NMR (101 MHz, DMSO-d₆): δ 67,2 ppm (d, *J*= 19,5 Hz, 2P).
¹³C-NMR (63 MHz, DMSO-d₆): δ 170,5 (m, 1C, Tr), 167,5 (m, 1C, Tr), 167,2 (m, 1C, Tr), 149,0 (d, *J =* 2,4 Hz, 1C), 148,8 (d, *J =* 2,3 Hz, 1C), 135,5 (s, 1C, Ph), 133,0 (t, *J =* 2,2 Hz, 2C), 132,2 (s, 1C, Ph), 131,4 (s, 2C), 130,9 (d, *J =* 49,2 Hz, 2C-P), 130,4 (m, 2C), 129,5 (s, 2C), 128,4 (s, 2C, Ph), 128,0 (s, 2C, Ph), 127,4 (d, *J =* 13,3 Hz, 2C), 125,6 (s, 2C), 123,8 (s, 2C), 123,5 (d, *J=* 5,7 Hz, 2C), 123,4 (s, 2C), 120,5 ppm (s, 2C).

¹H-NMR (250 MHz, DMSO-d₆): δ 8,97 (d, *J* = 9,7 Hz, 2H, 2NH-P), 8,35 (d, *J* = 6,8 Hz, 2H), 8,12 (d, *J=* 7,7 Hz, 4H), 7,73-7,63 (m, 4H), 7,63-7,47 (m, 5H), 7,39-7,17 (m, 4H), 7,06 ppm (d, *J* = 6,4 Hz, 2H).

IR (KBr): v 206 (m, N-H), 1540 (vs, O=C-N-H), 1506, 1486, 1424 (vs, P-Ph), 1197 (m, P-O-Ph), 1103, 943, 845, 879, 764 und 746 (s, C-H bend).

HRMS (EI) ber. für [12C₃₃H₂₃N₅P₂O₂]⁺: 583,1327, gef.: 583.1379 [M]+.
Elementaranalyse für C₃₃H₂₃N₅P₂O₂ (583,52 g/mol)
ber.: C 67,93, H 3,97, N 12,00, P 10,62%;
gef.: C 67,90, H 3,93, N 12,13, P 10,64%.

### Stufe 2 - Oxidation von DOP₂-PhGuanamin zu DOPO₂-PhGuanamin

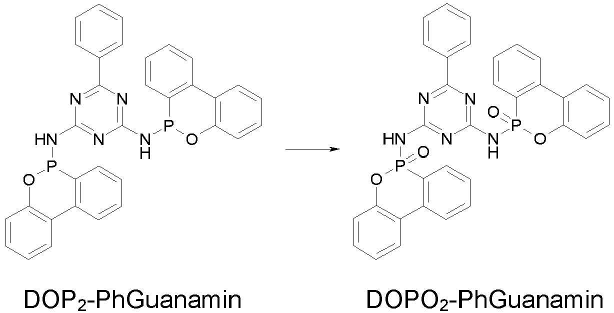

In einen mit Rückflusskühler, Rührvorrichtung, Thermometer und Tropftrichter ausgerüsteten Dreihalskolben wurden 11,68 g (0,0020 mol, 1 Äqu.) (DOP)₂-PhGuanamin in 50 ml Chloroform auf 40 °C erwärmt. Nach 15 min wurde das Gemisch mit einem Kühlbad auf ca. 20 °C gekühlt. Dann wurden unter kräftigem Rühren 2,88 g (0,048 mol, 2,2 Äqu.) einer 30%igen wässrigen Lösung von tert-Butylhydroperoxid langsam zugetropft. Das kräftige Rühren wurde 1 h lang fortgesetzt, wonach weitere 2,00 g (0,033 mol, 1,5 Äqu.) des Oxidationsmittels zugetropft wurden. Danach wurde noch 1 h lang bei Raumtemperatur gerührt und das Gemisch anschließend filtriert. Der isolierte Feststoff wurde mit Aceton gewaschen. Eine zusätzliche Fraktion des Produkts wurde erhalten, indem die Restlösung im Vakuum eingeengt wurde. Die vereinigten Fraktionen wurden in unter kräftigem Rühren in Aceton aufgeschlämmt, die Suspension wurde filtriert und der Feststoff im Luftstrom getrocknet. Auf diese Weise wurden 11,75 g (0,0019 mol, 95,0 % d.Th.) (DOPO)₂-PhGuanamin als weißes Pulver erhalten.
Fp.: 267-273 °C (Zers.)
³¹P-NMR (101 MHz, DMSO-d₆): δ 6,8 (s, 1P), 6,7 ppm (s, 1P).
¹³C-NMR (63 MHz, DMSO-d₆): δ 170,3 (s, 1C, Tr), 165,2 (t, *J =* 3,5 Hz, 2C, Tr), 149,7 (d, *J* = 7,4 Hz, 1C), 149,6 (d, *J =* 7,5 Hz, 1C), 135,5 (d, *J* = 7,3 Hz, 2C), 134,1 (s, 1C, Ph), 133,1 (d, *J* = 0,9 Hz, 2C), 132,1 (s, 1C, Ph), 130,5 (s, 2C), 130,3 (m, 2C), 128,5 (d, *J* = 15,1 Hz, 2C), 127,8 (s, 2C, Ph), 127,7 (s, 2C, Ph), 125,2 (s, 2C), 124,5 (s, 2C), 123,8 (d, *J* = 164,3 Hz, 1C-P), 123,7 (d, *J* = 164,3 Hz, 1C-P), 123,5 (d, *J* = 11,5 Hz, 2C), 120,9 (d, *J* = 12,0 Hz, 1C), 120,8 (d, *J=* 12,2 Hz, 1C), 120,0 ppm (m, 2C).

¹H-NMR (250 MHz, DMSO-d₆): δ 10,39 (t, *J =* 8,0 Hz, 2H, 2NH-P), 8,36-8,28 (m, 4H), 8,11-7,88 (m, 2H), 7,70 (t, *J=* 7,4 Hz, 2H), 7,57-7,16 (m, 9H), 7,07-6,87 ppm (m, 4H).

IR (KBr): v 3179 (w, N-H), 1587 (C=C), 1538 (vs, O=C-N-H), 1493, 1454, 1417 (vs, P-Ph), 1232 (s, P=O), 1205 (s, P-O-Ph), 1119, 1088, 943, 876, 751 und 785 (s, C-H bend).

HRMS (EI) ber. für [12C₃₃H₂₃N₅P₂O₄]⁺: 615,1225, gef.: 615,1290 [M]+.

Elementaranalyse für C₃₃H₂₃N₅P₂O₄ (615,51 g/mol)
ber.: C 64,39, H 3,77, N 11,38, P 10,06%;
gef.: C 64,07, H 3,78, N 11,25, P 10,16%.

### Beispiel 3

### Herstellung von DOPO-Mel und DOPO₂-Mel

### Stufe 1 - Herstellung von DOP-Mel und DOP₂-Mel

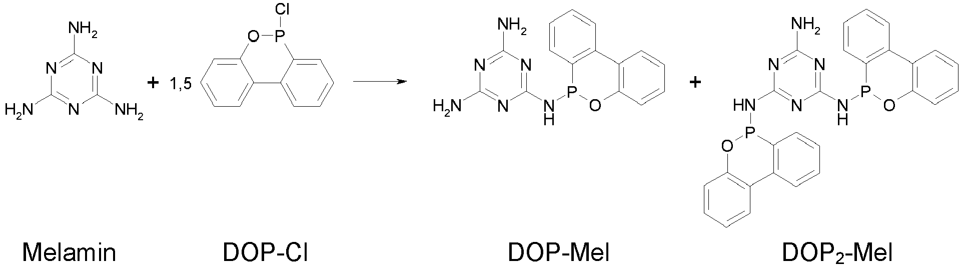

In einen mit Argon gefüllten 250-ml-Dreihalsrundkolben, der mit einem Innenthermometer, Tropftrichter, Rührer sowie einer Inertgasüberleitung ausgerüstet war, wurden 6,31 g (0,050 mol) Melamin sowie 50 g (ca. 0,6 mol) wasserfreies 1-Methylimidazol gefüllt und auf 100 °C erhitzt. Dann wurden 17,6 g (0,075 mol) DOP-CI bei 100 °C unter Inertbedingungen geschmolzen und in den Tropftrichter gefüllt. Das DOP-CI wurde unter kräftigem Rühren im Verlauf von 45 min bei 100 °C zugetropft, wobei ein Heißluftföhn benutzt wurde, um es flüssig zu halten. Nach Beendigung des Zutropfens wurde 3 h lang bei 120 °C und anschließend 1 h lang bei 145 °C gerührt. Das ³¹P-NMR-Spektrum der so erhaltenen Lösung zeigte, dass DOP-Mel und (DOP)₂-Mel als Reaktionsprodukte entstanden waren. Diese Lösung wurde nach Abkühlen auf ca. 60 °C ohne Aufarbeitung in der zweiten Stufe eingesetzt.

### Stufe 2 - Oxidation von DOP-Mel und DOP₂-Mel zu DOPO-Mel und DOPO₂-Mel

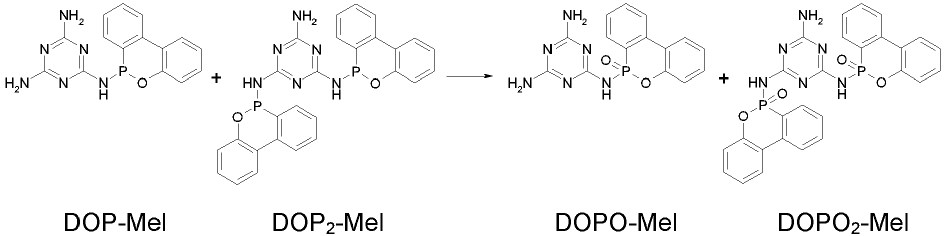

Zu dem in Stufe 1 erhaltenen Gemisch aus DOP-Mel und (DOP)₂-Mel in 1-Methylimidazol wurden 20 g (ca. 0,08 mol) einer ca. 37%igen Toluol-Lösung von tert-Butylhydroperoxid bei 60-65 °C binnen 45 min unter Rühren zugetropft. Nach Beendigung der Reagenszugabe wurde die Temperatur 2 h lang bei ca. 70 °C gehalten. Dann wurde das Reaktionsgemisch in 300 ml Wasser mit einer Temperatur von 50 °C eingerührt. Nach dem Abkühlen wurde dekantiert. Die so erhaltene Substanz wurde 15 h lang im Luftstrom getrocknet, im Mörser zerkleinert und danach 20 min lang in 300 ml Ethanol unter Rückfluss gerührt. Nach dem Abkühlen auf ca. 50 °C wurde filtriert und der Feststoff bei 90 °C im Vakuum vorgetrocknet. Schließlich wurde er ca. 10 h lang im Vakuumtrockenschrank auf 170 °C erhitzt (Druck ca. 12 mbar). Die ¹H-sowie ³¹P-NMR-Spektren des Produkts zeigten einen Gesamtgehalt von etwa 98 % an DOPO-Mel und (DOPO)₂-Mel in einem Molverhältnis von rund 1:1.

³¹P-NMR (101 MHz, DMSO-d₆): δ 8,79 ppm (DOPO-Mel); 7,60; 7,42 ppm ((DOPO)₂-Mel).
¹H-NMR (250 MHz, DMSO-d₆): δ 9,8-9,1 (NH-P); 8,15-8,03; 7,8-7,6; 7,55-7,45; 7,45-7,25; 7,25-7,2; 6,8-5,7 (NH₂).
MS (ESI): 341 (DOPO-Mel, M+1); 555 ((DOPO)₂-Mel, M+1).

### Beispiel 4

Herstellung von DPhPO₂-PhGuanamin, d.h. 2,4-Bis(diphenylphosphoryl)-6-phenyl-1,3,5-triazin:

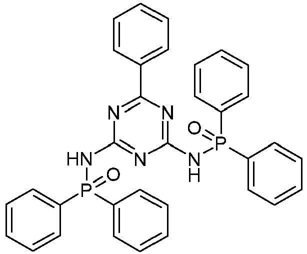

### Stufe 1 - Herstellung von DPhP₂-PhGuanamin aus Phenylguanamin und DPhP-Cl

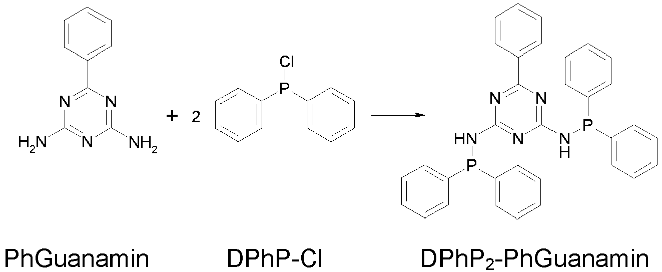

In einen mit Argon gefüllten Dreihalskolben, der mit Rückflusskühler, Thermometer, Rührvorrichtung sowie einem Tropftrichter ausgerüstet war, wurden Phenylguanamin (2,38 g, 0,0127 mol, 1 Äqu.), 1-Methylimidazol (2,29 g, 0,0279 mol, 2,2 Äqu.) sowie 40 ml wasserfreies Toluol gefüllt und auf 80 °C erhitzt. Bei dieser Temperatur wurden unter Rühren 6,16 g (0,0279 mol, 2,2 Äqu.) Diphenylphosphinylchlorid (DPHP-Cl) langsam zugetropft. Danach wurde das nun zweiphasige Reaktionsgemisch 2 h lang bei 90 °C gerührt. Danach wurde die obere Phase unter inerten Bedingungen vom zähflüssigen Bodenkörper (1-Methylimidazoliumchlorid) dekantiert und dabei über ein Verbindungsstück in einen zweiten, ebenfalls mit Argon gefüllten Dreihalskolben übergeführt. Auf diese Weise wurde eine Lösung von DPhP₂-PhGuanamin erhalten, die ohne Aufarbeitung in der zweiten Stufe eingesetzt wurde.

### Stufe 2 - Oxidation von DPhP₂-PhGuanamin zu DPhPO₂-PhGuanamin

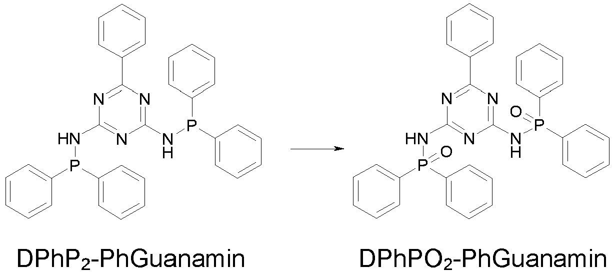

Der Dreihalskolben mit der in Stufe 1 hergestellten Lösung von DPhP₂-PhGuanamin wurde mit einem Rückflusskühler, Thermometer, Rührer sowie einem Tropftrichter ausgerüstet, der mit 6,86 g (0,0381 mol, 3 Äqu.) einer 11 %igen Lösung von H₂O₂ in Essigsäureethylester befüllt wurde. Dann wurde das Reaktionsgemisch mit einem Eiswasserbad auf ca. 5 °C gekühlt und das Oxidationsmittel langsam und unter kräftigem Rühren zugetropft, wobei die Temperatur bei maximal 15 °C gehalten wurde. Nach Beendigung der H₂O₂-Zugabe wurde das Kühlbad entfernt und das Reaktionsgemisch weitere 15 h lang gerührt. Der ausgefallene Feststoff wurde abfiltriert, mit Toluol gewaschen und danach 1,5 h lang in 50 ml siedendem Toluol gerührt. Anschließend wurde die heiße Suspension filtriert, und der Feststoff wurde erneut mit Toluol gewaschen und schließlich im Vakuum getrocknet (20 h, 150 °C). Auf diese Weise wurden 5,64 g (0,0096 mol, 75,6 % d.Th.) DPhPO₂-PhGuanamin als weißer Feststoff erhalten.

³¹P-NMR (101 MHz, DMSO-d₆): δ 16,1 ppm.
¹H-NMR (250 MHz, DMSO-d₆): δ 9,89 (d, *J =* 10,2 Hz, 2H, 2NH), 7,85 (m, 8H), 7,50 ppm (m, 14H), 7,15 ppm (m, 3H).
HRMS (EI) ber. für [12C₃₃H₂₇N₅P₂O₂]+: 586,1562; gef.: 586,1656 [M-H]+.

### Beispiel 5 - Messungen der Flammschutzwirkung

Ein Epoxynovolak, D.E.N. 438 von Dow Chemicals mit einem EEW (Epoxyäquivalentgewicht) von 179 g/mol, wurde mit 0,1 Gew.-% Triethanolamin und der neuen Verbindung der Erfindung, DOPO₃-Mel, als Flammschutzadditiv - in der zur Einstellung des erforderlichen Phosphorgehalts im Prüfkörper jeweils notwendigen Menge - vermischt. Das Gemisch wurde anschließend 2 h lang bei 140 °C gehalten, mittels Vakuum entgast und auf 90 °C abgekühlt. Die so hergestellte Präformulierung wurde bei 90 °C mit 6 Gewichtsteilen Dicyandiamid und 2 Gewichtsteilen Fenuron, bezogen auf 100 Gewichtsteile Epoxynovolak, vermischt. Die Aushärtung erfolgte in einem Aluminiumschälchen durch vorsichtiges Erhitzen auf 120°C innerhalb von 30 min, Halten dieser Temperatur für 1 h, Erhöhung der Temperatur auf 130 °C für 1 h und anschließendes Halten einer Temperatur von 200 °C für 2 h. Daraus wurden Probenkörper mit 70 x 13 x 4 mm geformt und zur Charakterisierung der Brandeigenschaften gemäß UL94 klassifiziert.

Bei UL94 handelt es sich um die Prüfvorschrift der Underwriters Laboratories, die inhaltsgleich in die IEC/DIN EN 60695-11-10 und -20 übernommen wurde. Dabei wirken Zündflammen mit einer Leistung von 50 W zweimal kurzzeitig auf den Probenkörper ein, wobei bei der Vertikalprüfung die Brennzeit und das Abfallen brennender Teile mithilfe eines unterhalb des Probenkörpers angeordneten Wattebauschs bewertet werden. Die Klassifizierung erfolgt in den Stufen "V0", "V1" und "V2", die in nachstehender Tabelle 1 erläutert werden:

**Tabelle 1: UL94-Klassifizierung**

| Klassifizierung | **V0** | **V1** | **V2** |
|---|---|---|---|
| Nachbrennzeit nach jeder Beflammung | ≤ 10 s | ≤ 30 s | ≤ 30 s |
| Gesamtbrenndauer je Satz (10 Beflammungen) | ≤ 50 s | ≤ 250 s | ≤ 250 s |
| Nachbrennzeit/Nachglühen nach der 2. Beflammung | ≤ 30 s | ≤ 60 s | ≤ 60 s |
| Abbrand bis zur Haltekammer | nein | nein | nein |
| Entzündung der Watte | nein | nein | ja |

Die Klassifizierung "V0" stellt demnach die höchste Anforderung beim Brandschutz dar und ist daher beim Einsatz von Flammschutzzusammensetzungen anzustreben. In der nachstehenden Tabelle 2 sind die Ergebnisse der Prüfung für DOPO₃-Mel sowie für DOPO als Vergleichssubstanz angegeben.

**Tabelle 2**

| **Flammschutzadditiv** | **Phosphorgehalt (Gew.-%)** | **UL94-Klassifizierung** |
|---|---|---|
| - | 0,0 | nicht klassifiziert |
| Vergl.: DOPO | 1,0 | nicht klassifiziert |
| Vergl.: DOPO | 1,4 | V1 |
| Vergl.: DOPO | 1,6 | V0 |
| Erf.: DOPO₃-Mel | 1,0 | V1 |
| Erf.: DOPO₃-Mel | 1,4 | V0 |

Aus diesen Ergebnissen geht eindeutig eine verbesserte Flammschutzwirkung von DOPO₃-Mel gegenüber dem handelsüblichen Additiv DOPO hervor. Somit eignet sich die neue Verbindung DOPO₃-Mel sehr gut als Flammschutzmittel in Kunststoffen.

## Patentansprüche

1. Verfahren zur Herstellung von Verbindungen der nachstehenden Formel (I) 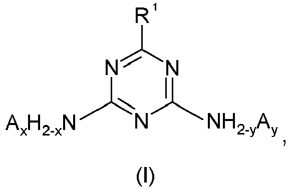 worin:
- der Rest R¹ aus -NH₂, -NH_{2-z}A_{z} sowie einwertigen Alkyl- und Arylresten ausgewählt ist,
- die Reste A jeweils unabhängig voneinander aus den nachstehenden Phosphoryl-Resten DOPO-, DPhPO- und DPhOPO- ausgewählt sind 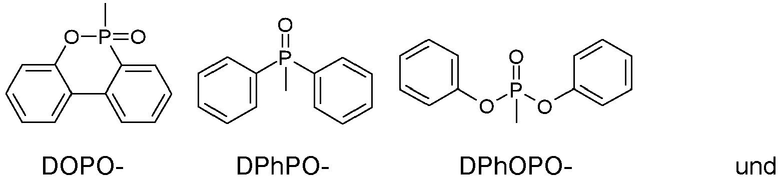
- die Indizes x, y, und z jeweils unabhängig voneinander für 0 oder 1 stehen, wobei zumindest einer der Indizes ≠ 0 ist;
indem in einer ersten Stufe Melamin oder, wenn R¹ ein Alkyl- oder Arylrest ist, das entsprechende Alkyl- oder Arylguanamin mit einem oder mehreren der nachstehenden Phosphinylchloride DOP-Cl, DPhP-Cl und DPhOP-Cl 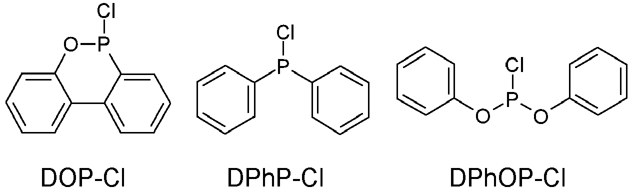 umgesetzt wird, um einen oder mehrere Phosphinylrest(e) an die Aminogruppe(n) des Melamins oder Guanamins zu binden, wonach in einer zweiten Stufe der/die gebundene(n) Phosphinylrest(e) durch Reaktion mit einem Oxidationsmittel zu dem/den entsprechenden Phosphorylrest(en) oxidiert wird/werden.

2. Verfahren nach Anspruch 1, **dadurch gekennzeichnet, dass** der einwertige Alkyl- oder Arylrest von R¹ aus -CH₃ und -C₆H₅ ausgewählt ist.

3. Verfahren nach Anspruch 1 oder 2, **dadurch gekennzeichnet, dass** als Oxidationsmittel ein Peroxid eingesetzt wird.

4. Verfahren nach Anspruch 3, **dadurch gekennzeichnet, dass** als Oxidationsmittel Wasserstoffperoxid oder t-Butylhydroperoxid eingesetzt wird.

5. Verfahren nach einem der vorangegangenen Ansprüche, **dadurch gekennzeichnet, dass** die erste Stufe in Gegenwart eines Säurefängers durchgeführt wird.

6. Verfahren nach Anspruch 5, **dadurch gekennzeichnet, dass** der Säurefänger gleichzeitig als Lösungsmittel fungiert.

7. Verfahren nach Anspruch 6, **dadurch gekennzeichnet, dass** 1-Methylimidazol als Säurefänger und Lösungsmittel eingesetzt wird.

8. Verfahren nach einem der vorangegangenen Ansprüche, **dadurch gekennzeichnet, dass** die zweite Stufe in einem organischen Lösungsmittel, ausgewählt aus Chloroform und Toluol durchgeführt wird.

9. Verfahren nach einem der vorangegangenen Ansprüche, **dadurch gekennzeichnet, dass** die erste Stufe bei einer Temperatur im Bereich von 100 bis 200 °C durchgeführt wird.

10. Verfahren nach einem der vorangegangenen Ansprüche, **dadurch gekennzeichnet, dass** die zweite Stufe bei einer Temperatur im Bereich von 50 bis 100 °C durchgeführt wird.

11. 2,4,6-Tris(9,10-dihydro-9-oxa-10-oxo-10-phosphaphenanthren-10-ylamino)-1,3,5-triazin (DOPO₃-Mel): 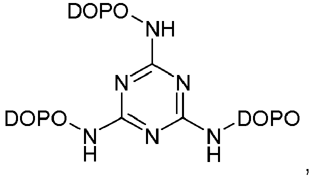 worin DOPO- dem Rest 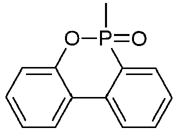 entspricht.

12. Verwendung von 2,4,6-Tris(9,10-dihydro-9-oxa-10-oxo-10-phosphaphenanthren-10-ylamino)-1,3,5-triazin als Flammschutzmittel.

## Claims

1. A method for producing compounds with the following formula (I): 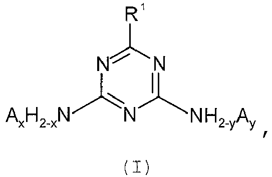 wherein:
- the residue R¹ is selected from -NH₂, -NH_{2-z}A_{z} as well as monovalent alkyl and aryl residues,
- the residues A are each selected, independently of each other, from the following phosphoryl residues DOPO-, DPhPO- and DPhOPO-: 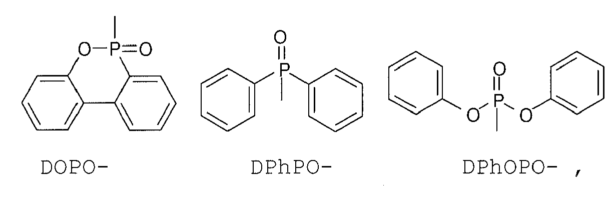 and
- the indices x, y and z each, independently of each other, represent 0 or 1, wherein at least one of the indices 0;
in which, in a first step, melamine or, when R¹ is an alkyl or aryl residue, the corresponding alkyl or aryl guanamine is reacted with one or more of the following phosphinyl chlorides DOP-Cl, DPhP-Cl and DPhOP-Cl: 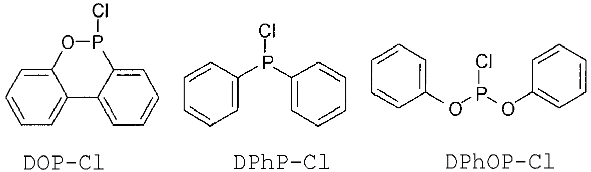 in order to bind one or more phosphinyl residue (s) to the amino group(s) of the melamine or guanamine, after which in a second step, the bound phosphinyl residue(s) is(are) oxidized by reaction with an oxidizing agent to form the corresponding phosphoryl residue(s).

2. The method as claimed in claim 1, **characterized in that** the monovalent alkyl or aryl residue of R¹ is selected from -CH₃ and -C₆H₅.

3. The method as claimed in claim 1 or claim 2, **characterized in that** a peroxide is used as the oxidizing agent.

4. The method as claimed in claim 3, **characterized in that** hydrogen peroxide or t-butyl hydroperoxide is used as the oxidizing agent.

5. The method as claimed in one of the preceding claims, **characterized in that** the first step is carried out in the presence of an acid scavenger.

6. The method as claimed in claim 5, **characterized in that** the acid scavenger simultaneously functions as a solvent.

7. The method as claimed in claim 6, **characterized in that** 1-methylimidazole is used as the acid scavenger and solvent.

8. The method as claimed in one of the preceding claims, **characterized in that** the second step is carried out in an organic solvent selected from chloroform and toluene.

9. The method as claimed in one of the preceding claims, **characterized in that** the first step is carried out at a temperature in the range 100°C to 200°C.

10. The method as claimed in one of the preceding claims, **characterized in that** the second step is carried out at a temperature in the range 50°C to 100°C.

11. 2,4,6-tris(9,10-dihydro-9-oxa-10-oxo-10-phosphaphenanthrene-10-ylamino)-1,3,5-triazine (DOPO₃-mel): 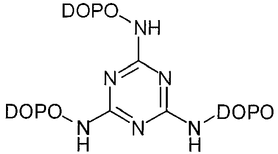 wherein DOPO- corresponds to the residue

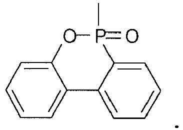

12. Use of 2,4,6-tris(9,10-dihydro-9-oxa-10-oxo-10-phosphaphenanthrene-10-ylamino)-1,3,5-triazine as a flame retardant.

## Revendications

1. Procédé destiné à préparer des composés de la formule (I) suivante : 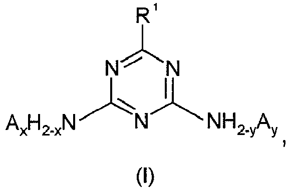 dans laquelle
- le radical R¹ est choisi parmi -NH₂, -NH_{2-z}A_{z}, ainsi que des radicaux alkyle ou aryle monovalents,
- les radicaux A chaque fois indépendamment les uns des autres sont choisis parmi les radicaux phosphoryle suivants DOPO-, DPhPO et DPhOPO 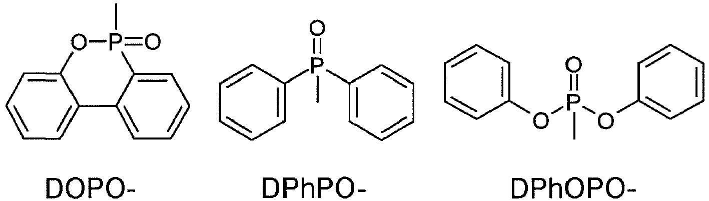 et
- les indices x, y et z, chaque fois indépendamment les uns des autres signifient 0 ou 1, au moins l'un des indices étant ≠ 0 ;
lors duquel, dans une première étape, on transforme de la mélamine ou si R¹ est un radical alkyle ou aryle, un guanamine alkyle ou un guanamine aryle correspondant avec un ou plusieurs des chlorures de phosphinyle suivants DOP-CI, DPhP-CI et DPhOP-CI 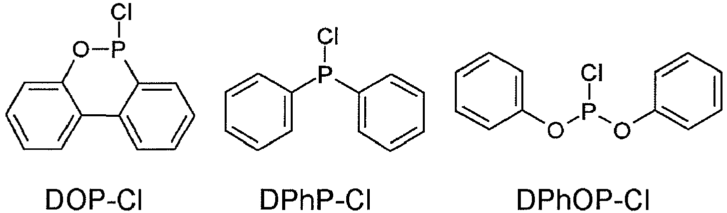 pour lier un ou plusieurs radical (radicaux) phosphinyle au(x) groupe(s) amino de la mélamine ou du guanamine, suite à quoi, dans une deuxième étape, on fait oxyder le radical (les radicaux) phosphinyle liés par réaction avec un agent oxydant pour obtenir le(s) radical (radicaux) phosphinyle correspondant(s).

2. Procédé selon la revendication 1, **caractérisé en ce que** le radical alkyle ou aryle monovalent est choisi parmi -CH₃ et C₆H₅.

3. Procédé selon la revendication 1 ou la revendication 2, **caractérisé en ce qu'**en tant qu'agent oxydant, on met en oeuvre un peroxyde.

4. Procédé selon la revendication 3, **caractérisé en ce qu'**en tant qu'agent oxydant, on met en oeuvre un peroxyde d'hydrogène ou un t-butylhydroperoyde.

5. Procédé selon l'une quelconque des revendications précédentes, **caractérisé en ce que** la première étape est réalisée en présence d'un capteur d'acide.

6. Procédé selon la revendication 5, **caractérisé en ce que** le capteur d'acide fait simultanément office de solvant.

7. Procédé selon la revendication 6, **caractérisé en ce qu'**en tant que capteur d'acide et de solvant, on met en oeuvre un 1-méthylimidazole.

8. Procédé selon l'une quelconque des revendications précédentes, **caractérisé en ce que** la deuxième étape est réalisée dans un solvant organique, choisi parmi le chloroforme et le toluène.

9. Procédé selon l'une quelconque des revendications précédentes, **caractérisé en ce que** le première étape est réalisée à une température de l'ordre de 100 à 200 °C.

10. Procédé selon l'une quelconque des revendications précédentes, **caractérisé en ce que** la deuxième étape est réalisée à une température de l'ordre de 50 à 100 °C.

11. 2,4,6-tris(9,10-dihydro-9-oxa-10-oxo-10-phosphaphénanthrène-10-ylamino)-1,3,5-triazine (DOPO₃-mel) : 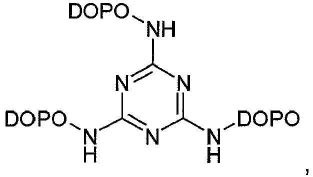 dans laquelle DOPO correspond au radical

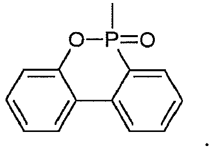

12. Utilisation de 2,4,6-tris(9,10-dihydro-9-oxa-10-oxo-10-phosphaphénanthrène-10-ylamino)-1,3,5-triazine en tant qu'agent pare-flamme.
